(19) Europäisches Patentamt
European Patent Office
Office européen des brevets

(11) Numéro de publication: **0 156 734**
**A2**

(12) # DEMANDE DE BREVET EUROPEEN

(21) Numéro de dépôt: **85400579.0**

(22) Date de dépôt: **26.03.85**

(51) Int. Cl.⁴: **C 07 D 487/04**
A 61 K 31/50, C 07 D 237/20
C 07 D 237/12
//(C07D487/04, 249:00, 237:00)

(30) Priorite **30.03.84 FR 8405086**

(43) Date de publication de la demande:
**02.10.85 Bulletin 85/40**

(84) Etats contractants désignés:
**AT BE CH DE FR GB IT LI LU NL SE**

(71) Demandeur: **SANOFI, société anonyme**
**40, Avenue George V**
**F-75008 Paris(FR)**

(72) Inventeur: **Bourguignon, Jacques**
**2 rue Feldwasser**
**F-67150 Hipsheim(FR)**

(72) Inventeur: **Chambon, Jean-Pierre**
**Chemin des Truquets Route de Montpellier**
**F-34570 Montarnaud(FR)**

(72) Inventeur: **Wermuth, Camille-Georges**
**3 rue de la Côte d'Azur**
**F-67100 Strasbourg(FR)**

(74) Mandataire: **Gillard, Marie-Louise et al,**
**Cabinet Beau de Loménie 55, Rue d'Amsterdam**
**F-75008 Paris(FR)**

(54) **Triazolo4,3-bpyridazines, procédé pour leur préparation et compositions pharmaceutiques les contenant.**

(57) La présente invention concerne des triazolo [4,3-*b*] pyridazines de formule:

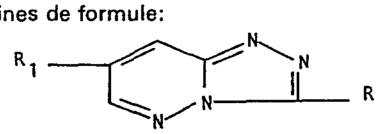

(I)

dans laquelle R représente l'hydrogène, un groupe alkyle inférieur ou un groupe phényle, un groupe chlorophényle ou un groupe benzyle et $R_1$ représente un groupe phényle, ou halogénophényl ou trifluorométhyl phényle ou alkyle inférieur phényle ou alcoxy inférieur phényle ou encore un groupe phénylalkyle inférieur; ou un de ses sels pharmaceutiquement acceptables.

Ces composés conviennent comme antagonistes des benzodiazépines.

EP 0 156 734 A2

Triazolo[4,3-b]pyridazines, procédé pour leur préparation et compositions pharmaceutiques les contenant.

La présente invention concerne des triazolo [4 ,3-b] pyridazines portant en position 7 un groupe phényle éventuellement substitué ou un groupe phénylalkyle inférieur, un procédé pour leur préparation et des compositions pharmaceutiques contenant lesdites triazolopyridazines comme ingrédients actifs.

Des triazolo [4,3-b]pyridazines portant en position 6 un groupe phényle éventuellement substitué sont connues en littérature.

La demande de brevet de la République Fédérale d'Allemagne 2 741 763 décrit des 6-phényl-1,2,4-triazolo [4,3-b] pyridazines portant éventuellement dans les positions 3, 7 et 8 des groupes alkyle.

La demande de brevet de la République Fédérale d'Allemagne 3 217 325 décrit également des 6-phényl-1,2,4-triazolo [4,3-b]pyridazines qui sont cependant substituées dans la position 3 par un groupe amino libre.

Les composés des deux demandes de brevet ci-dessus possèdent une activité anxiolytique, sédative et anticonvulsivante démontrée, pour les composés de la demande de brevet de la République Fédérale d'Allemagne 2 741 763, en comparaison avec une benzodiazépine, à savoir le chlordiazépoxyde.

On a maintenant trouvé, d'une façon inattendue, que certaines triazolo[4,3-b]pyridazines portant en position 7 un groupe phényle éventuellement substitué ou un groupe phénylalkyle inférieur antagonisent les effets des benzodiazépines et sont donc utiles comme antidotes dans l'abus des benzodiazépines et des composés agissant sur les récepteurs des benzodiazépines.

Ainsi, la présente invention a pour objet, selon un de ses aspects, des 1,2,4-triazolo [4,3-b]-pyridazines substituées en position 7, de formule :

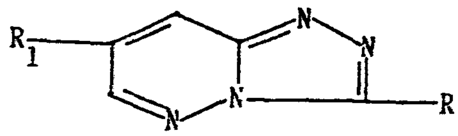

(I)

dans laquelle R représente l'hydrogène, un groupe alkyle inférieur, un groupe phényle, un groupe chlorophényle ou un groupe benzyle et $R_1$ représente un groupe phényle, un phényle substitué par un atome d'halogène, un phényle substitué par un groupe trifluorométhyle, un phényle substitué par un alkyle inférieur, un phényle substitué par un alcoxy inférieur, ou encore un groupe phénylalkyle inférieur; ainsi que leurs sels pharmaceutiquement acceptables.

Le terme "alkyle inférieur", tel qu'utilisé ici, désigne le radical d'un hydrocarbure aliphatique saturé contenant jusqu'à 6 atomes de carbone, tel que méthyle, éthyle, n-propyle, n-hexyle, isobutyle.

Le terme "alcoxy inférieur" désigne un hydroxyle dont l'atome d'hydrogène est remplacé par un groupe alkyle inférieur, tel que défini ci-dessus.

Selon un autre de ses aspects, la présente invention a pour objet un procédé pour la préparation des 7-phényl-1,2,4-triazolo[4,3-b]pyridazines de formule (I) ci-dessus et de leurs sels pharmaceutiquement acceptables, caractérisé en ce que l'on utilise comme produit de départ un composé de formule :

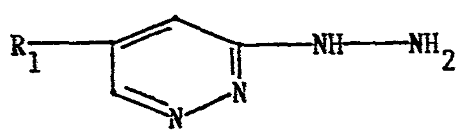

(II)

dans laquelle $R_1$ est tel que défini ci-dessus. Suivant une première variante du procédé, on traite le produit (II) par un orthoester de formule :

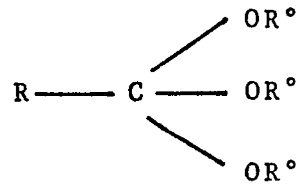

dans laquelle R est tel que défini ci-dessus et R° représente un groupe méthyle ou éthyle, dans un solvant organique à la température de 50 à 175°C et, éventuellement, on transforme le produit ainsi obtenu en ses sels pharmaceutiquement acceptables.

Comme solvant organique on utilise de préférence un alcool, tel que le méthanol, l'éthanol, l'isopropanol, le n-butanol, le n-pentanol et similaires.

Selon un mode opératoire préférentiel, après un chauffage de 5-9 heures dans du n-butanol à reflux, la réaction est terminée et le produit final est isolé sous forme de base libre par simple filtration et purifié selon les techniques connues.

Selon une seconde variante, on traite l'hydrazine (II) par l'aldéhyde RCHO pour former l'hydrazone correspondante qui, par chauffage en présence d'un agent d'oxydation : l'azidodicarboxylate de diéthyle, conduit au produit (I).

La formation de l'hydrazone est effectuée par chauffage des réactifs dans un solvant convenable : chlorure de méthylène, méthanol ou mélange des 2 solvants, à la température du reflux. L'hydrazone brute obtenue par évaporation à siccité du mélange réactionnel est dissoute dans le n-butanol, additionnée d'azidodicarboxylate de diéthyle en excès, et chauffée au reflux pendant 3 à 10 heures. On isole le produit sous forme de base libre par évaporation du solvant et on le purifie selon les techniques connues.

Les composés de départ de formule (II) sont préparés à partir d'une 3-pyridazinone substituée en position 5 de formule :

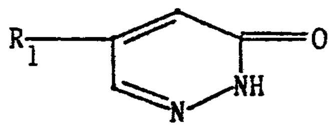

(III)

dans laquelle $R_1$ est tel que défini ci-dessus, par réaction avec l'oxychlorure de phosphore; après un traitement avec un hydroxyde alcalin, de sodium de préférence, on isole la 3-chloropyridazine de formule :

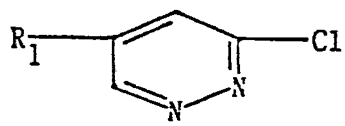

(VI)

où $R_1$ est tel que défini ci-dessus, et l'on traite le produit ainsi obtenu avec l'hydrate d'hydrazine en excès à une température de 60 à 90°C sous atmosphère inerte pendant une période de 4 à 8 heures. Le produit de formule(II) est isolé selon les méthodes conventionnelles

Les composés de formules(II) et (IV) sont nouveaux et représentent un objet ultérieur de la présente invention.

Les composés de formule (III) utilisés comme matières premières dans le procédé de la présente invention sont en général nouveaux; ils peuvent être préparés selon la méthode décrite par J.J. BOURGUIGNON et C.G. WERMUTH (J. Org. Chem. 1981, 46, 4889-4894) pour la préparation de la 5-phénylpyridazine-3-one (formule II $R_1$ = phényle) en remplaçant le phénylacétaldéhyde par un acétaldéhyde $R_1$-substitué ($R_1$ : autre que phényle).

Les exemples suivants illustrent l'invention sans toutefois la limiter.

PREPARATION 1 :

a) - Méthyle 3-(4-méthoxyphényl)-2,3-époxy-propanoate.

A une solution de méthylate de sodium dans le méthanol (obtenue à partir de 3,5 g de sodium et 50 ml de méthanol) refroidie à -10°C, on ajoute 13,6 g de p-méthoxybenzaldéhyde puis goutte à goutte 10,85 g de chloracétate de méthyle. On agite pendant 2 heures à -5°C puis pendant 3 heures à température ambiante.

On verse le mélange réactionnel sur 200 g de glace contenant 1,15 ml d'acide acétique. On essore le solide, lave avec de l'eau et sèche sous vide. Poids : 16 g.

b) - Sodium 3-(4-méthoxyphényl)-2,3-époxy propionate.

Au mélange de 13,5 g du produit obtenu en a) et 82 ml de benzène, on ajoute lentement à +5°C la solution de méthylate de sodium dans le méthanol obtenue à partir de 1,54 g de sodium et 21,5 ml de méthanol. On ajoute ensuite 1,36 ml d'eau puis 60 ml d'éther. On laisse reposer puis essore le solide. Après séchage, on obtient 12,3 g du produit attendu.

c) - (4-méthoxyphényl)acétaldéhyde.

A 10 g du sel de sodium obtenu ci-dessus dans 50 ml de benzène, on ajoute 2,8 g d'acide acétique et porte au reflux pendant 2 heures 30.

Après refroidissement, on ajoute 100 ml de benzène et 15 ml d'eau. On sépare la couche organique, lave avec de l'eau, sèche la solution et évapore à siccité. On obtient ainsi 5 g du produit attendu.

d) 4-hydroxy 3-(4-méthoxyphényl)-3-butène-4-olide.

A la solution de 3,22 g d'acide glyoxylique et 4,3 g de chlorhydrate de morpholine dans 30 ml de dioxanne, on ajoute 5 g de 4-(méthoxyphényl)acétaldéhyde. On agite le mélange pendant 1 heure à température

ambiante puis on chauffe 24 heures au reflux. On évapore le solvant et reprend le résidu dans l'eau. On essore le solide et sèche sous vide. On obtient 6,2 g du produit attendu.

Chromatographie en couche mince : Rf = 0,67 (acétate d'éthyle-hexane, 1-1 vol/vol).

e) - 5-(4-méthoxyphényl)-2H-pyridazine-3-one.

On chauffe au reflux pendant 1 heure 30 le mélange de 4,1 g du produit obtenu ci-dessus et 1,1 g d'hydrate d'hydrazine dans 60 ml d'éthanol.

On laisse reposer puis on essore le solide et sèche. On obtient 2,82 g du produit attendu.

Chromatographie en couche mince : Rf = 0,15 (acétate d'éthyle-hexane, 1-1 vol/vol).

PREPARATIONS 2 à 8 :

En opérant comme décrit dans la préparation 1, on obtient :

- la 5-(4-chlorophényl)-2H-pyridazine-3-one (2) ;
- la 5-(3-chlorophényl)-2H-pyridazine-3-one (3) ;
- la 5-(2-chlorophényl)-2H-pyridazine-3-one (4) ;
- la 5-(3-trifluorométhylphényl)-2H-pyridazine-3-one (5) ;
- la 5-(4-méthylphényl)-2H-pyridazine-3-one (6) ;
- la 5-benzyl-2H-pyridazine-3-one (7) ;
- la 5-(2-phényléthyl)-2H-pyridazine-3-one (8).

EXEMPLE I :

a) - 3-chloro-5-(4-méthoxyphényl)pyridazine.

On chauffe à 80°C, sous agitation, pendant 4 heures, 2,7 g de 5-(4-méthoxyphényl)-2H-pyridazine-3-one et 19,9 g d'oxychlorure de phosphore.

On verse ensuite lentement sous agitation le mélange dans l'eau glacée puis on alcalinise avec une solution de soude à 20%. On essore le précipité, lave avec beaucoup d'eau puis sèche. On obtient ainsi 2,4 g du produit attendu.

Chromatographie en couche mince : Rf = 0,2 (acétate d'éthyle-hexane, 1/3 vol/vol).

b) - 3-hydrazino-5-(4-méthoxyphényl)-pyridazine.

On chauffe à 80°C sous atmosphère d'azote pendant 6 heures le mélange de 2,3 g du produit obtenu ci-dessus et 13,6 g d'hydrate d'hydrazine.

On ajoute de l'eau et essore le solide qui s'est séparé. Après séchage, on obtient 2,8 g du produit attendu.

Spectre de RMN : enregistré en solution dans le deutero chlorforme.

3 H à 3,8 ppm (singulet) - 3H à 4,3 ppm (singulet élargi) - 4H à 7,0-7,5 ppm (système AB, J AB = 9 Hz) - 1H à 7,1 ppm (doublet) - 1H à 8,7 ppm (doublet, J = 2 Hz)

EXEMPLES II à IX :

Par réaction des composés décrits dans les préparations 2 à 8 et de 5-phényl-2H-pyridazin -3-one (J. Org. Chem. 1981, 46, 4893) avec l'oxychlorure de phosphore comme décrit dans l'exemple I a) et traitement des produits ainsi obtenus comme décrit dans l'exemple I b), on obtient :

EXEMPLE II :

a) - 3-chloro-5-(4-chlorophényl)pyridazine.

b) - 5-(4-chlorophényl)-3-hydrazinopyridazine.

EXEMPLE III :

a) - 3-chloro-5-(3-chlorophényl)pyridazine.

b) - 5-(3-chlorophényl)-3-hydrazinopyridazine.

EXEMPLE IV :

a) - 3-chloro-5-(2-chlorophényl)pyridazine.

b) - 5-(2-chlorophényl)-3-hydrazinopyridazine.

EXEMPLE V :

a) - 3-chloro-5-(3-trifluorométhylphényl)pyridazine.

b) - 3-hydrazino-5-(3-trifluorométhylphényl)pyridazine.

EXEMPLE VI :

a) - 3-chloro-5-(4-méthylphényl)pyridazine.

b) - 3-hydrazino-5-(4-méthylphényl)pyridazine.

EXEMPLE VII :

a) 3-chloro-5-benzyl pyridazine.

b) 5-benzyl-3-hydrazinopyridazine.

EXEMPLE VIII :

a) 3-chloro-5-(phényl-2 éthyl)pyridazine.

b) 3-hydrazino-5-(phényl-2 éthyl)pyridazine.

EXEMPLE IX :

a) - 3-chloro-5-phényl pyridazine.

b) - 3-hydrazino-5-phényl pyridazine.

Exemple 1 :

7-(4-méthoxyphényl)-3-méthyl-1,2,4-triazolo[4,3-b]-pyridazine. (SR 95287).

On chauffe au reflux pendant 7 heures le mélange de 1,9 g de 3-hydrazino-5-(4-méthoxyphényl)pyridazine, 2,85 g d'orthoacétate d'éthyle dans 13,7 ml de n-butanol.

Après refroidissement, on essore les cristaux qui se sont séparés et sèche sous vide. On obtient 1,43 g du produit attendu ; F : 228°C.

Exemple 2 :

7-phényl-1,2,4-triazolo[4,3-b]pyridazine. (SR 95194)

On opère comme dans l'exemple 1 à partir de 3-hydrazino-5-phényl pyridazine et d'orthoformiate d'éthyle ; F : 180°C.

En opérant comme dans l'exemple 1 mais en faisant varier la 3-hydrazino pyridazine utilisée comme produit de départ, on obtient les produits (I) rassemblés dans le tableau I .

TABLEAU I

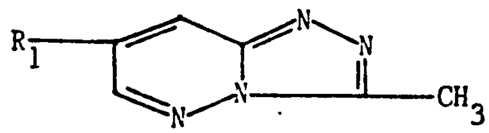

| Exemple No. | Produit No.SR | $R_1$ | Point de fusion (solvant) |
|---|---|---|---|
| 3 | 95 195 | 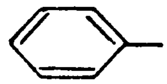 | 216°C (n-butanol) |
| 4 | 95 252 | 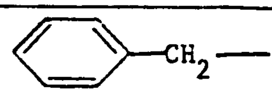 | 114°C (n-butanol) |
| 5 | 95 410 | 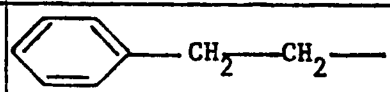 | 142°C (isopropanol) |
| 6 | 95 370 | 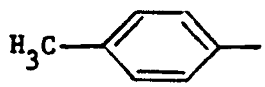 | 196°C (n-butanol) |
| 7 | 95 437 | 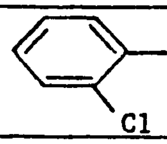 | 198°C (acétate d'éthyle) |
| 8 | 95 398 | 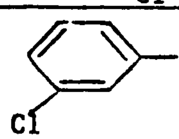 | 200°C (éthanol) |
| 9 | 95 369 | 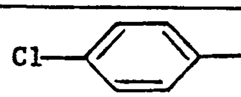 | 238°C (éthanol) |
| 10 | 95 533 | 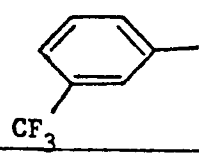 | 188°C (éthanol) |

Exemple 11 :

7-phényl-3-benzyl-1,2,4-triazolo [4,3-b]-pyridazine. (SR 95 491)

On mélange la solution de 5 g de 3-hydrazino-5-phényl pyridazine dans 150 ml de dichlorométhane avec la solution de 3,5 ml de phénylacétaldéhyde dans 100 ml de méthanol, puis on chauffe à 60°C pendant 3 heures.

On évapore à siccité sous vide, puis on reprend le résidu dans 250 ml de n-butanol, et on ajoute 5,5 ml d'azidodicarboxylate de diéthyle. On chauffe au reflux pendant 7 heures puis on évapore le solvant sous vide.

On purifie le résidu par chromatographie sur colonne pour obtenir 2,5 g du produit attendu ; F = 140°C.

En opérant comme dans l'exemple 11, mais en remplaçant le phénylacétaldéhyde par du benzaldéhyde ou du chlorobenzaldéhyde, on obtient les produits (I) réunis dans le Tableau II.

TABLEAU II

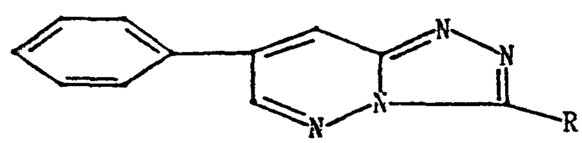

| Exemple No. | Produit No. SR | R | Point de fusion (solvant) |
|---|---|---|---|
| 12 | 95 494 | 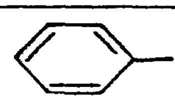 | 210°C (isopropanol) |
| 13 | 95 492 | 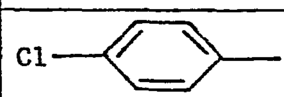 | 222°C (éthanol) |

Les composés selon l'invention ont été étudiés en ce qui concerne leurs propriétés thérapeutiques.

Les 1,2,4-triazolo [4,3-b] pyridazines substituées en position 7 de la présente invention antagonisent, tant in vitro qu'in vivo, les effets des benzodiazépines.

Aptitude des dérivés à déplacer le flunitrazépam tritié du récepteur des benzodiazépines.

Les études ont été conduites sur des préparations synaptiques de cerveau de rat selon la méthode décrite par Squives et Braestrup (Nature, 1977, 266, pages 732 à 734) et Möhler et Okada (Science, 1977, 198, pages 849 à 851).

Le flunitrazépam tritié (60 Ci/mM) a été utilisé à la concentration finale de 1,1 nM. Les résultats obtenus avec divers produits de l'invention figurent dans le tableau III et sont exprimés en concentration efficace médiane ($IC_{50}$) en µM.

TABLEAU III

| Produits No. SR | $IC_{50}$ |
|---|---|
| 95 195 | 9 |
| 95 252 | 27 |
| 95 398 | 2,6 |
| 95 194 | 49 |
| 95 533 | 2,9 |
| 95 491 | 4,5 |
| 95 494 | 50 |

Les produits selon l'invention sont aptes à déplacer le flunitrazépam tritié in vitro à des concentrations variant de 2,6 à 50 µM. Ils sont donc susceptibles de se lier au récepteur des benzodiazépines.

<u>Aptitude des dérivés à antagoniser les propriétés pharmacologiques des benzodiazépines.</u>

a) Propriétés myorelaxantes.

Les études ont été effectuées grâce au test de traction (J.R. Boissier et coll. Archives Internationales de Pharmacodynamie, 1961, 135, pages 29-49). Ce test détermine la capacité des souris, placées par les pattes antérieures sur un fil tendu horizontalement, à s' agripper par les pattes postérieures. Les animaux qui n'ont pas ramené les pattes postérieures sur le fil en moins de 5 secondes sont considérés en état de myorelaxation.

Les produits sont administrés 15 minutes avant l'injection par voie intrapéritonéale d'une dose de 3 mg/kg de diazépam et le test est pratiqué 60 minutes après l'administration du produit à étudier. L'étude a été réalisée sur des lots de 10 souris par dose.

Les résultats obtenus avec divers produits de l'invention figurent dans le tableau IV.

<u>TABLEAU IV</u>

| Produits No. SR | Antagonisme de la myorelaxation Dose efficace 50% ou effet (dose) |
|---|---|
| 95 195 | $ED_{50}$ = 26 mg/kg |
| 95 398 | $ED_{50}$ = 20 mg/kg |
| 95 533 | antagonisme 100% à 100 mg/kg |

b) Activité anxiolytique.

Les études ont été conduites chez le rat dans le test de conflit approche-évitement (L. Cook et A.B. Davidson, effects of behaviorally active drugs in a conflict-punishment procedure in rats. In : The Benzodiazepines, S. Garattini, E. Mussini et L.O. Randall Eds ; Raven Press, New York, 1973, 327-345).

Un produit représentatif de l'invention, SR 95 195, a été administré par voie intrapéritonéale 60 minutes avant une dose anxiolytique de diazépam (4 mg/kg i.p.). Le test a été effectué 30 minutes après administration du diazépam.

Les résultats obtenus en fonction de la dose de SR 95 195 administrée sont les suivants :

| Dose du produit | % d'antagonisme |
|---|---|
| 1,25 mg/kg | 26% |
| 6,25 mg/kg | 74% |
| 25 mg/kg | 100% |

ce qui traduit la capacité du produit à antagoniser l'effet anxiolytique du diazépam selon un effet proportionnel à la dose.

Les produits selon l'invention se caractérisent donc par leur aptitude à antagoniser l'effet myorelaxant et l'effet anxiolytique d'une benzodiazépine. Par contre, les 6-phényl-1,2,4-triazolo[4,3-b]pyridazines, décrites dans la demande de brevet de la République Fédérale d'Allemagne No. 2 741 763, testées dans les mêmes conditions, potentialisent les effets myorelaxants et anxiolytiques du diazépam.

Par ailleurs, les produits selon l'invention ont été étudiés en ce qui concerne leur aptitude à diminuer la consommation alimentaire du rat à jeun.

Les études ont été conduites chez des rats femelles (200 à 240 g) isolés et entraînés à consommer leur nourriture pendant une période de 4 heures par jour. Le jour du test les animaux reçoivent, soit le produit à étudier, soit le véhicule d'administration, 60 minutes avant la présentation de la nourriture. On détermine la quantité de nourriture ingérée pendant la première heure.

Dans le tableau ci-après figurent les résultats obtenus avec le composé SR 95 195 administré per os à la

dose de 40 mg/kg. Les valeurs qui y figurent sont les moyennes de groupes de 6 rats.

| Produit administré | Consommation de nourritures | |
|---|---|---|
| | en g | Variation % par rapport aux témoins |
| Véhicule (gomme arabique à 5%) | 17,6 ± 1,3 | |
| SR 95 195 | 8,6 ± 1,5 | - 51% ++ |

++ p < 0,01 test de Student

Enfin les produits de l'invention ont été étudiés en ce qui concerne leur toxicité.

Les études ont été effectuées sur des lots de 5 souris femelles Charles Rivers CD1 de poids compris entre 20 et 24 g. Les produits ont été administrés par voie orale, dilués dans la gomme arabique à 5%. La toxicité aigüe a été relevée pendant les 72 heures qui ont suivi l'administration du produit.

Les résultats suivants (pourcentage d'animaux morts) ont été obtenus en fonction de la dose administrée.

| Produit No. SR | % de toxicité | | |
|---|---|---|---|
| | 250 mg/kg | 500 mg/kg | 1000 mg/kg |
| 95 194 | 0 | 0 | 100 |
| 95 195 | 0 | 0 | 80 |
| 95 252 | 0 | 0 | 100 |
| 95 410 | 0 | 0 | 0 |
| 95 287 | 0 | 0 | 0 |
| 95 370 | 40 | 100 | 100 |
| 95 437 | 0 | 0 | - |
| 95 398 | 0 | 0 | - |
| 95 369 | 0 | 100 | 100 |
| 95 442 | 0 | 0 | - |
| 95 494 | 0 | 0 | - |
| 95 492 | 0 | 0 | - |

Les doses létales de ces dérivés sont considérablement plus élevées que leurs doses actives dans les tests décrits précédemment.

Les produits selon l'invention présentent des propriétés pharmacologiques intéressantes et une faible toxicité. Par suite ils peuvent être utilisés en thérapeutique humaine pour le traitement d'affections psychiques, neurologiques ou neuromusculaires.

En particulier, ils peuvent être utilisés pour le traitement des troubles de la vigilance et du comportement alimentaire. Ils peuvent être également utilisés comme antidotes en cas d'intoxications aux benzodiazépines. Ils peuvent aussi être associés à des produits antiparasitaires afin de supprimer leurs effets dépresseurs sur le système nerveux central.

La présente invention a donc pour objet, selon un autre de ses aspects, des compositions pharmaceutiques contenant comme ingrédients actifs les composés de formule (I).

Les compositions pharmaceutiques de la présente invention peuvent être présentées sous les formes convenant à l'administration en thérapie, telles que comprimés, dragées, capsules, gélules, poudres, granules, suspensions ou sirops pour l'administration orale, de suppositoires pour l'administration rectale, de solutions stériles pour l'administration parentérale.

La quantité de principe actif peut varier entre 1 et 250 mg de principe actif par unité de dosage selon que cette unité est destinée à l'administration orale, parentérale ou rectale, mais ladite quantité peut atteindre jusqu'à 1000 mg dans le cas de préparation pour infusion intraveineuse lente.

Dans les unités de dosage, l'ingrédient actif peut être seul ou en mélange avec les véhicules ou excipients pharmaceutiques conventionnels tels que, par exemple,

eau distillée, solution aqueuse pour préparations injectables, amidon, talc, stéarate de magnésium, polyvinylpyrrolidone, acide alginique, silice colloïdale, chlorure de sodium, dioxyde de titane, beurre de cacao, agents édulcorants et similaires.

A titre d'exemple de composition pharmaceutique, on prépare des gélules à base d'un des composés des exemples 1 à 13 ayant la composition suivante :

| | |
|---|---|
| Principe actif | 50 mg |
| Lactose | 85 mg |
| Stéarate de magnésium | 5 mg |

en mélangeant intimement des charges des ingrédients ci-dessus et en versant le mélange dans des gélules de gélatine dure.

## REVENDICATIONS

1. 1,2,4-triazolo [4,3-b] pyridazines substituées en position 7, de formule :

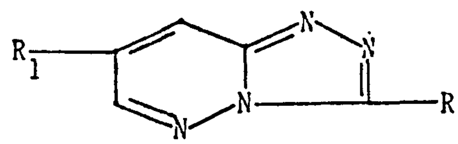

(I)

dans laquelle R représente l'hydrogène, un groupe alkyle inférieur ou un groupe phényle, un groupe chlorophényle ou un groupe benzyle et $R_1$ représente un groupe phényle, ou halogénophényl ou trifluorométhyl phényle ou alkyle inférieur phényle ou alcoxy inférieur phényle ou encore un groupe phénylalkyle inférieur; ou un de ses sels pharmaceutiquement acceptables.

2. 7-phényl-3-méthyl-1,2,4-triazolo[4,3-b]pyridazine ou un de ses sels pharmaceutiquement acceptables.

3. 7-(3- chlorophényl )-3-méthyl-1,2,4-triazolo[4,3-b] pyridazine ou un de ses sels pharmaceutiquement acceptables.

4. 7-(3-trifluorométhyl)-3-méthyl-1,2,4-triazolo-[4,3-b] pyridazine ou un de ses sels pharmaceutiquement acceptables.

5. 7-phényl-3-benzyl-1,2,4-triazolo[4,3-b]pyridazine ou un de ses sels pharmaceutiquement acceptables.

6. Procédé de préparation d'une 1,2,4-triazolo-[4,3-b]pyridazine substituée en position 7 selon la revendication 1, caractérisé en ce que l'on traite un composé de formule :

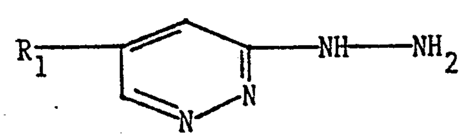

(II)

dans laquelle $R_1$ est tel que défini dans la revendication 1, avec un orthoester de formule :

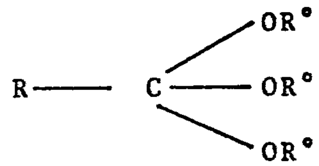

dans laquelle R est tel que défini dans la revendication 1 et R° représente un groupe méthyle ou éthyle, dans un solvant organique à la température de 50 à 175°C et l'on transforme, si nécessaire, le produit ainsi obtenu en un de ses sels pharmaceutiquement acceptables.

7. Procédé de préparation d'une 1,2,4-triazolo-[4,3-b]pyridazine substituée en position 7 selon la revendication 1, caractérisé en ce que l'on traite un composé de formule :

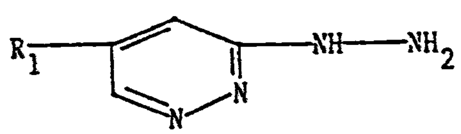 (II)

dans laquelle $R_1$ est tel que défini dans la revendication 1 avec un aldéhyde de formule : R-CHO dans laquelle R est tel que défini dans la revendication 1, dans un solvant organique à température de 40 à 80°C pour former l'hydrazone correspondante, et que l'on cyclise cette dernière par chauffage à une température de 100 à 150°C dans un solvant organique en présence d'un agent d'oxydation l'azido dicarboxylate de diéthyle, et que l'on transforme, si nécessaire, le produit ainsi obtenu en un de ses sels pharmaceutiquement acceptables.

8. Procédé selon l'une des revendications 6 ou 7, caractérisé en ce que le composé (II) est obtenu par traitement de la 3-chloropyridazine de formule :

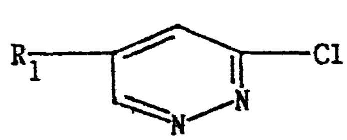 (IV)

dans laquelle $R_1$ est tel que défini dans la revendication 1, avec de l'hydrate d'hydrazine en excès à une température de 60 à 90°C sous atmosphère inerte.

9. A titre d'intermédaires de synthèse utiles pour l'obtention des composés de la revendication 1, les composés de formule :

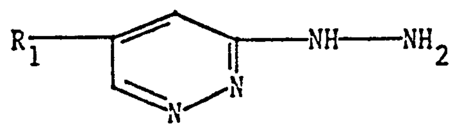

(II)

dans laquelle $R_1$ est tel que défini dans la revendication 1.

10. 3-hydrazino-5-phényl pyridazine.

11. A titre d'intermédiaires de synthèse utiles pour l'obtention des composés de la revendication 1, les composés de formule

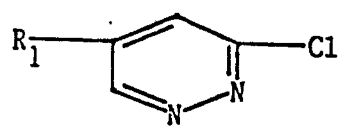

(IV)

dans laquelle $R_1$ est tel que défini dans la revendication 1.

12. Composition pharmaceutique à action antagoniste des benzodiazépines, caractérisée en ce qu'elle contient, en tant qu'ingrédient actif, un composé selon une des revendications 1 à 5.

13. Composition selon la revendication 10, caractérisée en ce qu'elle contient de 1 à 1000 mg de principe actif par unité de dosage en mélange avec un excipient ou véhicule pharmaceutique.

03/MLG/CB
H 2233 CAS 310

## REVENDICATIONS POUR L'ETAT CONTRACTANT : AT

1. Procédé pour l'obtention de 1,2,4-triazolo-[4,3-b]pyridazines substituées en position 7 et répondant à la formule :

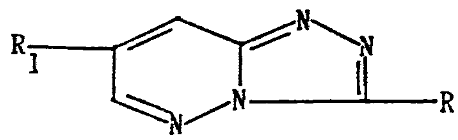

(I)

dans laquelle R représente l'hydrogène, un groupe alkyle inférieur ou un groupe phényle, un groupe chlorophényle ou un groupe benzyle et $R_1$ représente un groupe phényle, ou halogénophényl ou trifluorométhyl phényle ou alkyle inférieur phényle ou alcoxy inférieur phényle ou encore un groupe phénylalkyle inférieur ; ou un de ses sels pharmaceutiquement acceptables, caractérisé en ce que l'on traite un composé de formule :

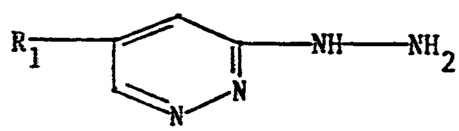

(II)

dans laquelle $R_1$ est tel que défini ci-dessus avec un orthoester de formule :

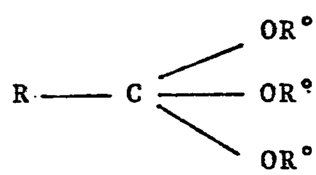

dans laquelle R est tel que défini ci-dessus et R° représente un groupe méthyle ou éthyle, dans un solvant organique à la température de 50 à 175°C et l'on transforme, si nécessaire, le produit ainsi obtenu en un de ses sels pharmaceutiquement acceptables.

2. Procédé pour l'obtention de 1,2,4-triazolo-/¯4,3-b7pyridazines substituées en position 7 et répondant à la formule :

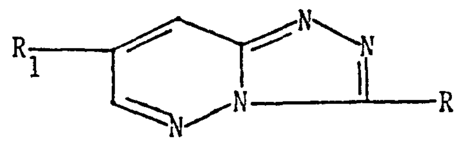

(I)

dans laquelle R représente l'hydrogène, un groupe alkyle inférieur ou un groupe phényle, un groupe chlorophényle ou un groupe benzyle et $R_1$ représente un groupe phényle, ou halogénophényl ou trifluorométhyl phényle ou alkyle inférieur phényle ou alcoxy inférieur phényle ou encore un groupe phénylalkyle inférieur ; ou un de ses sels pharmaceutiquement acceptables, caractérisé en ce que l'on traite un composé de formule :

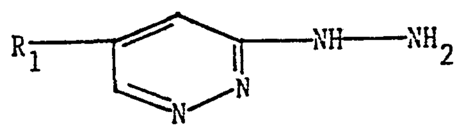

(II)

dans laquelle $R_1$ est tel que défini ci-dessus avec un aldéhyde de formule : R-CHO dans laquelle R est tel que défini ci-dessus dans un solvant organique à température de 40 à 80°C pour former l'hydrazone correspondante, et que l'on cyclise cette dernière par chauffage à une température de 100 à 150°C dans un solvant organique en présence d'un agent d'oxydation l'azido dicarboxylate de diéthyle, et que l'on transforme, si nécessaire, le produit ainsi obtenu en un de ses sels pharmaceutiquement acceptables.

3. Procédé selon l'une des revendications 1 ou 2, caractérisé en ce que le composé de formule (II) est obtenu par traitement d'une 3-chloropyridazine de formule :

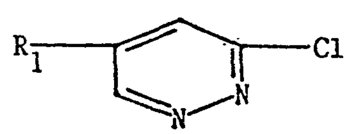

(IV)

dans laquelle $R_1$ est tel que défini dans la revendication 1, avec un excès d'hydrate d'hydrazine à une température de 60 à 90°C sous atmosphère inerte.

4. Procédé selon la revendication 3, caractérisé en ce que le composé de formule IV est obtenu à partir d'une 3-pyridazinone substituée en position 5 de formule :

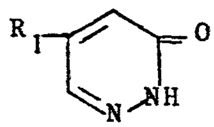

dans laquelle $R_1$ est tel que défini dans la revendication 1, par réaction avec l'oxychlorure de phosphore et traitement avec un hydroxyde alcalin.

5. Utilisation de 1,2,4-triazolo[4,3-b] pyridazines substituées en position 7 et répondant à la formule :

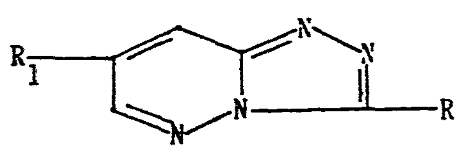

(I)

dans laquelle R représente l'hydrogène, un groupe alkyle inférieur ou un groupe phényle, un groupe chlorophényle ou un groupe benzyle et $R_1$ représente un groupe phényle, ou halogénophényl ou trifluorométhyl phényle ou alkyle inférieur phényle ou alcoxy inférieur phényle ou encore un groupe phénylalkyle inférieur ; ou un de ses sels pharmaceutiquement acceptables pour la préparation de médicaments à action antagoniste des benzodiazépines.